(19) Europäisches Patentamt
European Patent Office
Office européen des brevets

(11) EP 0 776 903 B1

(12) **EUROPEAN PATENT SPECIFICATION**

(45) Date of publication and mention of the grant of the patent:
**29.01.2003 Bulletin 2003/05**

(51) Int Cl.[7]: **C07H 13/04**, C07H 13/06, C07H 13/08

(21) Application number: **96308584.0**

(22) Date of filing: **27.11.1996**

(54) **Process for the production of sucrose-6-ester.**

Herstellungsverfahren für Sucrose-6-Ester

Procédé de préparation de sucrose-6-ester

(84) Designated Contracting States:
**AT BE CH DE DK ES FR GB GR IE IT LI LU MC NL PT SE**

(30) Priority: **28.11.1995 US 7660**

(43) Date of publication of application:
**04.06.1997 Bulletin 1997/23**

(73) Proprietor: **McNeil-PPC, Inc.**
**Skillman, NJ 08558-9418 (US)**

(72) Inventors:
- **White, James A.**
  **Athens, GA 30605 (US)**
- **Bradford, Billy A.**
  **Athens, GA 30605 (US)**

(74) Representative: **Fisher, Adrian John**
**CARPMAELS & RANSFORD**
**43 Bloomsbury Square**
**London WC1A 2RA (GB)**

(56) References cited:
**EP-A- 0 352 048** **EP-A- 0 475 619**

- **PATENT ABSTRACTS OF JAPAN vol. 011, no. 297 (C-448), 25 September 1987 & JP 62 087248 A (KURARAY CO LTD), 21 April 1987,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 776 903 B1

## Description

The invention relates to an improvement in a process for the regioselective esterification of sucrose utilizing a distannoxane diester as a catalyst.

### Background of the Invention

The artificial sweetener 4,1',6'-trichloro-4,1',6'-trideoxygalactosucrose ("sucralose") is derived from sucrose by replacing the hydroxyls in the 4, 1', and 6' positions with chlorine. (In the process of making the sweetener, the stereo configuration at the 4 position is reversed - hence the compound is a galactosucrose.) The direction of the chlorine atoms to only the desired positions is a major synthesis problem because the hydroxyls that are replaced are of differing reactivity; two are primary and one is secondary. The synthesis is further complicated by the fact that the primary hydroxyl in the 6 position is unsubstituted in the final product.

A number of different synthetic routes for the preparation of sucralose have been developed in which the reactive hydroxyl in the 6 position is first blocked, as by an ester group, prior to the chlorination of the hydroxyls in the 4, 1', and 6' positions, followed by hydrolysis to remove the ester substituent to produce sucralose. Several of such synthesis routes involve tin-mediated syntheses of sucrose-6-esters. Illustrative are the tin-mediated routes disclosed by Navia (U.S. Patent No. 4,950,746), Neiditch et al. (U.S. Patent No. 5,023,329), and Walkup et al. (U.S. Patent No. 5,089,608).

In EP A 0 475 619, published on March 18, 1992, and also in United States Patent No. 5,470,969 (a continuation-in-part of the United States application on which EP A 0 475 619 was based), an improved process for producing sucrose-6-ester is disclosed. This process comprises reacting sucrose with a carboxylic acid anhydride in a reaction mixture comprising a polar aprotic solvent and a 1,3-diacyloxy-1,1,3,3-tetra(hydrocarbyl)distannoxane ("distannoxane diester" or "DSDE"), for a period of time and at a temperature sufficient to produce a sucrose-6-ester. In one important aspect, the DSDE-catalyzed process comprises:

(1) preparing a first reaction mixture comprising sucrose, a polar aprotic solvent, a second solvent capable of removing water by codistillation, and a 1,3-diacyloxy-1,1,3,3-tetra(hydrocarbyl)distannoxane;

(2) subjecting said first reaction mixture to codistillation to remove water, to form thereby a second reaction mixture; and

(3) adding a carboxylic acid anhydride to said second reaction mixture to form a third reaction mixture and maintaining said third reaction mixture at a temperature and for a period of time sufficient to produce a sucrose-6-ester.

This invention involves an improvement on this aspect of the DSDE-catalyzed process.

### Brief Summary of the Invention

Broadly, the invention provides a process which comprises passing vapors of a solvent capable of removing water by codistillation through a reaction mixture containing (a) a polar aprotic solvent and (b) sucrose and a 1,3-diacyloxy-1,1,3,3-tetra(hydrocarbyl)distannoxane and/or the reaction product of sucrose and a 1,3-diacyloxy-1,1,3,3-tetra (hydrocarbyl)distannoxane, in sufficient quantity and for a period of time sufficient to remove substantially all of the water in said reaction mixture by codistillation.

In one aspect, the invention provides a process which comprises:

(1) preparing a first reaction mixture comprising sucrose and a polar aprotic solvent, and optionally heating said first reaction mixture by passing vapors of a second solvent capable of removing water by codistillation through said first reaction mixture;

(2) adding a 1,3-diacyloxy-1,1,3,3-tetra(hydrocarbyl)-distannoxane to said first reaction mixture to form a second reaction mixture;

(3) passing vapors of a second solvent capable of removing water by codistillation through said second reaction mixture, in sufficient quantity and for a period of time sufficient to remove substantially all of the water in said reaction mixture by codistillation, to form thereby a third reaction mixture comprising said polar aprotic solvent and said second solvent, said second reaction mixture being substantially free of water, and;

(4) adding a carboxylic acid anhydride to said third reaction mixture to form a fourth reaction mixture and maintaining said fourth reaction mixture at a temperature and for a period of time sufficient to produce a sucrose-6-ester.

Detailed Description of the Invention

The process of the invention is an improvement in one important aspect of the process whereby sucrose is reacted with a carboxylic acid anhydride in a reaction mixture comprising a polar aprotic solvent and a distannoxane diester, for a period of time and at a temperature sufficient to produce a sucrose-6-ester. In the present process, (a) a first reaction mixture is prepared which comprises sucrose and a polar aprotic solvent. The first reaction mixture is optionally heated by passing vapors of a second solvent capable of removing water by codistillation through the first reaction mixture. In a second step, (b), DSDE is added to the first reaction mixture to form a second reaction mixture, (c) vapors of a second solvent (such as cyclohexane) capable of removing water by codistillation is passed through the second reaction mixture in sufficient quantity and for a period of time sufficient to remove water, to form a third reaction mixture comprising polar aprotic solvent and the second solvent, the resulting third reaction mixture being substantially free of water, and (d) a carboxylic acid anhydride is added to the third reaction mixture to form a fourth reaction mixture, which is maintained at a temperature and for a period of time sufficient to produce a sucrose-6-ester.

The stoichiometric ratio (mol:mol) of DSDE:sucrose is not narrowly critical. For instance, ratios ranging from about 0.10 to about 1.5 molar equivalents (basis sucrose) can be employed, producing sucrose-6-ester yields ranging from about 35% to about 80+%. Laboratory data show that, up to approximately a sucrose:DSDE ratio of about 1:1, sucrose-6-ester yields increase as the stoichiometric amount of DSDE is increased. Both 1,3-diacetoxy-1,1,3,3-tetrabutyldistannoxane (distannoxane diacetate or "DSDA") and 1,3-dibenzoyloxy-1,1,3,3-tetrabutyldistannoxane (distannoxane dibenzoate or "DSDB") have been shown to be effective for use in this process.

Polar aprotic solvents which have been employed include *N,N*-dimethylformamide ("DMF") and *N*-methyl-2-pyrrolidone. In a particularly preferred process, the polar aprotic solvent is DMF, and the DSDE is DSDA, DSDB, 1,3-diacetoxy-1,1,3,3-tetraoctyldistannoxane or 1,3-dibenzoyloxy-1,1,3,3-tetraoctyldistannoxane. Other suitable solvents include dimethyl sulfoxide, *N,N*-dimethylacetamide, and hexamethylphosphoramide.

Stoichiometric ratios of carboxylic acid anhydride ranging from about 1 to about 4 molar equivalents (basis sucrose) can be employed. Preferred stoichiometric ratios are in the range of from about 1.1 to about 1.8 molar equivalents. Stoichiometric ratios below about 1.1 molar equivalents can lead to an undesirable amount of unreacted sucrose in the final product, while ratios above about 1.8 can cause the formation of undesired sucrose diesters.

Acylation reaction temperatures ranging from about -10°C to about 60°C have been found to be useful. The particular reaction temperature employed is not a narrowly critical aspect of the invention, although acylation reaction temperature affects the rate of acylation and excessively high temperatures can increase the production of undesirable sucrose esters. Preferred acylation temperatures range from about 0°C to about 45°C. The acylation reaction will normally take from about 1/4 hour to about 6 hours at the indicated temperature range of 0° to 60°C.

Both acetic anhydride and benzoic anhydride have been shown to be effective acylating agents. Acetic anhydride is preferred, mostly for economic reasons and because distannoxane diacetate is preferred over distannoxane dibenzoate for use in the reaction with sucrose (this factor will be discussed in more detail below). A variety of other carboxylic acid anhydrides would be expected to function effectively in the practice of the invention. Examples of such other anhydrides are the anhydrides of substituted benzoic acid (e.g., 4-nitrobenzoic acid, 3,5-dinitrobenzoic acid, and the like), alkanoic acids such as propionic acid, butyric acid, cyclohexane-carboxylic acid, long chain fatty acids, both saturated and unsaturated, such as stearic acid, oleic acid, linoleic acid, and the like, having up to, for example, 28 carbon atoms, unsaturated acids such as acrylic acid and methacrylic acid, substituted acids such chloroacetic acid, cyanoacetic acid, phenoxyacetic acid, and the like.

The rate of acylation is dependent upon a number of variables, which include catalyst stoichiometry (increasing catalyst concentration relative to sucrose increases the rate of acylation), activity of the catalyst (e.g., DSDA appears to be a more active catalyst than DSDB), reactivity of the carboxylic acid anhydride (e.g., acetic anhydride is more reactive than benzoic anhydride), and the reaction temperature and the relative concentration of the reactive species (as the acylation is a multi-order process).

DSDE may be recovered for reuse by the method of Vernon et al., U.S. Patent No. 5,034,551. (In the present case, the DSDE may be used as recovered; Vernon et al. contemplated converting the DSDE to a distannoxane dialkoxide or a di(hydrocarbyl)tin oxide.) The acylation mixture is treated with a small amount of water and the DSDE is extracted in an essentially quantitative manner by contacting the mixture with a hydrocarbon such as toluene, cyclohexane, *n*-heptane, 2,2,4-trimethylpentane, or mixtures thereof, or an ether such as diethyl ether, di(*n*-propyl) ether, methyl *tert.*-butyl ether, or the like. Recycling the DSDE is advantageous for economic reasons because a large proportion of the tin species can be recovered for reuse, and for processing reasons because solids handling is reduced (i.e., the DSDE is recovered in solution).

After removal of the DSDE for recycle, the reaction mixture contains sucrose-6-ester, carboxylic acid (that

was formed by the reaction of carboxylic acid anhydride and sucrose plus any that might have been formed by the reaction of excess anhydride with the water that was added in the DSDE extraction step described above), some unreacted sucrose, a small amount of other sucrose esters, and polar aprotic solvent. It is preferred to remove the carboxylic acid from the solution of sucrose-6-ester in polar aprotic solvent prior to further processing of the sucrose-6-ester. This can be accomplished, for instance, by vacuum stripping when the acid is relatively volatile such as acetic acid. Make-up polar aprotic solvent may be added during the stripping operation, if it is desired to further process the sucrose-6-ester in the same solvent. (For example, if DMF is the solvent and chlorination via the Walkup et al. process will be the next step.) The desired sucrose-6-ester in residual polar aprotic solvent may then be used directly in subsequent processing, or optionally it may be recovered by conventional procedures such as crystallization from a solvent such as methanol. The carbohydrate impurities that are usually present (unreacted sucrose and other sucrose esters) do not have an adverse affect on the chlorination of the sucrose-6-ester to produce sucralose-6-ester.

Second solvents that are capable of codistillatively removing water include hydrocarbons, particularly saturated hydrocarbons, aromatic hydrocarbons, chlorinated hydrocarbons, ketones, and ethers. A very wide range of second solvents appear to be suitable for use in the invention. The primary criteria for a second solvent are that it produce a mixture with the polar aprotic solvent, the DSDE, and the sucrose, that refluxes with an internal reaction temperature within the range of from about 75°C to about 153°C (and preferably less than 100°C to minimize thermal degradation of sucrose), that it codistill the water present in the reaction mixture, and that it not render key reaction components (e.g., sucrose) insoluble.

Second solvents which are immiscible with water and which form a constant-composition minimum-boiling azeotrope with water are preferred, but the second solvent does not have to be capable of forming a constant-boiling azeotrope of constant composition with water to be effective for the use in the invention. Nor is it necessary that the second solvent be immiscible with water. It is necessary only that the second solvent be capable of codistilling the water from the reaction medium. The source of the water in the second reaction mixture is the small amounts of water present in the sucrose and polar aprotic solvent, and the water that is formed (by a reaction that has not been fully characterized) by interaction of the sucrose and DSDE. In the process of the invention, despite the interaction of the DSDE with sucrose, the DSDE is termed a "catalyst" because it is recoverable as DSDE at the end of the process.

It is postulated that the following sequence of reactions occurs in the process of the invention, using 1,3-diacetoxy-1,1,3,3-tetra(butyl)distannoxane (DSDA) for illustrative purposes as the distannoxane diester and acetic anhydride as the acylating agent:

Sucrose and DSDA react to give the sucrose/stannoxane adduct (1) and the dibutyltin hydroxide (2), as follows:

$$\underset{\text{(Sucrose)}}{\text{S-OH}} + \underset{\text{(DSDA)}}{\text{Bu}_2\text{Sn(OAc)-O-Sn(OAc)Bu}_2} \rightarrow$$

$$\underset{(1)}{\text{S-O-Sn(OAc)Bu}_2} + \underset{(2)}{\text{HO-Sn(OAc)Bu}_2}$$

Alkyltin hydroxides are known to dehydrate readily to yield distannoxanes[1]. As a result, 1 molar equivalent of (2) condenses to yield ½ molar equivalent each of water and DSDA. This is the source of the water generated by the reaction of sucrose with DSDE. Upon the completion of the reaction of sucrose with DSDA, the reaction mixture is cooled, the acylating agent is added, and the following reaction occurs:

$$\text{S-O-Sn(OAc)Bu}_2 + \text{Ac}_2\text{O} \rightarrow \text{S-6-A} + \text{Bu}_2\text{Sn(OAc)}_2$$

(1) + Acetic anhydride → sucrose-6-acetate +

dibutyltin diacetate

Dibutyltin diacetate is reported to hydrolyze in water to give DSDA and acetic acid[2]. Thus, if DSDA and acetic anhydride are used (as in the above illustrative series of reactions), the DSDA is recovered unchanged upon the

[1] A.J. Bloodworth and A.G. Davies, Chapter 4, in **Organotin compounds**, Ed. A.K. Sawyer, Marcel Dekker, NY 1971.
[2] M.G. Muralidhara and V. Chandrasekhar, **Ind. J. Chem.**, 1991, **30,** 487.

addition of water (quenching) to the reaction mixture after the acylation reaction is complete. If, for example, DSDA and benzoic anhydride are used, then one recovers a monobenzoyloxy, monoacetoxy dibutyl tin species (or its dimer, an analog of the original distannoxane diester).

Preferred second solvents for reasons of chemical stability, efficiency of water removal, cost, and boiling point include toluene, cyclohexane, *n*-heptane, and isooctane (2,2,4-trimethylpentane). The preferred dehydration temperature is within the range of from about 85°C to about 105°C. Temperatures below about 85°C can result in an unnecessarily slow dehydration, while temperatures greater than about 105°C can result in significant decomposition of the sucrose.

The major contribution of this invention is the step of carrying out the water removal step by passing (sparging) the second solvent, in the form of vapor, through the above-described second reaction mixture in sufficient quantity and for a period of time sufficient to remove water. Vapors of the second solvent are passed through the second reaction mixture to remove substantially all of the water from this reaction mixture. The water removal is continued until, for instance, the tin/water ratio is between about 20/1 to about 35/1, wt/wt. The tin measurement is % tin per se, not % DSDE, as measured by X-ray fluorescence, and the water is measured as % water by Karl-Fischer analysis.

The following experiments illustrate the invention:

<u>Equipment Layout</u>

The reactor system (pilot plant size) included an agitated, jacketed reactor connected to a circulation loop, cyclohexane boiler, vapor recovery system and solids feeder system. The circulation loop consists of a pump and heat exchanger. The cyclohexane boiler consists of a pressure vessel connected to a circulation loop, which has a pump and heat exchanger, and a vapor line which is connected through a sparger to the reactor. The vapor recovery system consists of a vapor condenser and a decant tank with heavy and light phase discharge pumps. The solids feeder system consists of a vibrated hopper and screw conveyer.

The flow of cyclohexane in the system is as follows; the cyclohexane is superheated to 20 psig in the boiler. Superheated cyclohexane vapors then enter the reactor through the sparger, contact the reaction mixture and the flow overhead to the condenser. (The vapors of cyclohexane are superheated in this case because the desired reaction temperature is higher than the normal boiling point of cyclohexane. The person skilled in the art will be able to determine the specific desired vapor temperature of the second solvent in individual cases, based on the reaction temperature desired, heat transfer rates, specific heat of the particular second solvent vapor, and other similar factors that are well known to chemical and process engineers.) The condensed vapors flow to the decant tank where the two immiscible phases [(1) water/DMF - heavy; (2) cyclohexane - light] are separated. The recovered cyclohexane is pumped to the cyclohexane boiler and the water/DMF is collected in a tote (i.e., a portable receiver for the condensed liquid).

<u>Test Run</u>

Cyclohexane was charged to the boiler and the circulation started. The reactor was charged with 390 kg of DMF and agitated. The DMF was heated, using 33% aqueous propylene glycol in the jacket as a heat exchange medium, to 45°C. 70 kg of sucrose was then charged to the reactor through the solids feed system. The reactor was heated to 85°C and the sucrose was allowed to dissolve. Once the sucrose was in solution the reactor was heated to 105°C (internal temperature) using the jacket.

The cyclohexane was superheated to 20 psig in the boiler. Once at temperature, the cyclohexane vapors were started to the reactor through the sparger. The cyclohexane vapors from the reactor were condensed, collected in the decant tank and pumped back to the boiler.

With the reactor at temperature and vapors flowing to the reactor, concentrated DSDA in cyclohexane (122.7 kg DSDA; 74.66 wt% DSDA/25.44 wt%, cyclohexane) at room temperature was added to the reactor over about 15 minutes, and the reaction time monitoring was started. During the DSDA addition, the temperature dropped to 94°C but recovered to the reaction temperature, 97°C, within 10 minutes. The concentrated DSDA line was then flushed with 51 kg of DMF. During the reaction the cyclohexane flow rate was maintained at about 1400 kg/hr and the reactor temperature at about 97°C. The interaction between sucrose and DSDA was taken to completion (monitored by the tin/water ratio) in 60 minutes. Owing to time delays in sampling and analyzing, the reaction was continued for 98 minutes. The cyclohexane boiler was then shut down, reactor agitation was started, and the reactor was cooled to 0°C using the 33% aq. propylene glycol cooled circulation loop.

When the reactor was at 0°C, 24.1 kg of acetic anhydride was charged over 15 minutes to the circulation loop of the reactor. The acetylation reaction was held at 0°C for a total of 360 minutes. At the end of the hold time, the solution was quenched with 21.7 kg of water and held for 30 minutes. The final carbohydrate profile was 86.01% S-6-A/S-2-A (Sucrose-6-Acetate/Sucrose-2-Acetate), 11.75% S-D-A (Sucrose-Di-Acetate), 0.59% S-M-A (other Sucrose-Mono-Acetates), and 1.65% sucrose.

The ratio of S-6-A/S-2-A (Sucrose-6-Acetate/Sucrose-2-Acetate) is typically about 18:1, corresponding to a yield of S-6-A alone of about 81.5% and S-2-A of 4.5%.

Alternate Method

Another method for the production of S-6-A is to dissolve the sucrose at 85°C then heat the solution to 105°C, and add the concentrated DSDA in about 15 minutes. During the DSDA addition the reactor temperature usually drops to about 92°C and requires heating back to reaction temperature of 97°C using the jacket. When the reactor is at temperature the cyclohexane vapors and reaction time monitoring are started. The course of the reaction is followed by monitoring the tin/water ratio. When the dehydration is complete, the reactor is cooled and acetylated at 0°C, then quenched as discussed above. This method, although recovering comparable product quality and yield, increases the sucrose/DSDE reaction time from 60 minutes to about 90 minutes and increases the total time the carbohydrates are exposed to elevated temperatures.

The primary difference in these two methods relates to overall reaction cycle time, which in turn is impacted by the heating sequence and regimen employed. In the first method, the introduction of cyclohexane vapors was employed to maintain the internal temperature during the organotin (DSDE) introduction. In the second method, the sparge system was turned on only after introduction of all of the reactants and equilibration at the appropriate temperature as achieved by jacket heating. The net result is that the latter heating regimen is slower. While this does not seem to adversely impact yield or quality, it does mean that fewer batches can be processed in a given time in the same equipment, and therefore equipment costs for fixed production volume correspondingly increase.

The Prior Art Method

Another method (the prior art method) for the production of S-6-A is to dissolve the sucrose at 85°C, heat to 105°C using the circulation loop and steam heating, and then adding the concentrated DSDA. During the DSDA addition the reactor temperature usually drops to about 90°C and requires heating back to reaction temperature of 97°C using the heat exchanger. The course of the reaction is followed by monitoring the tin/water ratio. When the dehydration is complete (by codistillation with cyclohexane, but using jacket heating rather than heating by sparging cyclohexane vapors through the reaction mixture), the reaction mixture is cooled and acetylated, held for three hours and quenched as discussed above. This method gives a product quality which usually contains about >3% residual sucrose, a slight reduction in yield, an increase in reaction time from 60 to 120 minutes and an increase in the total time the carbohydrates are exposed to elevated temperatures.

Due to the heat sensitivity and potential reversibility of the reaction between DSDE and sucrose by reaction of the DSDE-sucrose product with water, there is a need for gentle heating and efficient co-distillation to remove water. This need is met by the vigorous contacting of co-distillation solvent vapors being sparged into the second reaction mixture. Additionally, the time the reaction mixture is at elevated temperature determines the amount of carbohydrate degradation. By adjusting the order of addition (i.e., adding the DSDE after the sparging with the codistillation solvent vapors has begun), which allows the reaction to proceed while the reactor temperature is recovering after DSDA addition, and coupled with the sparged vapors concept, the time at elevated temperature is reduced significantly with the result that high yield is obtained on a more reproducible basis. With the prior art method, after DSDA addition, the system had to be reheated to reaction temperature before water removal could begin. This resulted in extended periods of time at elevated temperature.

Cyclohexane (or other co-distillation solvent) is heated in a boiler, and injected into the bottom of the reactor containing the sucrose/DMF solution. The reaction mixture is heated to 105°C by direct contact with the cyclohexane. The concentrated DSDA solution, at room temperature, is then added to the reactor over 15 minutes, which results in a drop in temperature to about 94°C. The reaction time monitoring starts when the DSDA charge starts. The reaction continues while the temperature increases back to 97°C. During the reaction, the cyclohexane vapors contact the reaction products, heating and co-distilling the water formed from the system. The water removal from the system is the rate limiting step and determines the overall reaction time.

The solution is then cooled to, e.g., 0°C and acetylated. Cold acetylation helps to assure that the amount of unreacted sucrose is kept to a minimum. Unreacted sucrose is undesirable because it directly affects the yield of S-6-A and it forms undesirable chlorinated compounds which are difficult to separate from the sucralose-6-A during subsequent processing. The overall acetylation time is preferably about six hours or more to allow the reaction to go to completion at the cold temperature.

After acetylation, the reaction mixture is quenched with water to hydrolyze all the acetic anhydride and to regenerate DSDE (presumably from dialkyltin diester that is present, as discussed above). This quench is carried out as described in EP A 0 475 619, published on March 18, 1992, and in United States Patent No. 5,470,969.

The advantages of the method of this invention are:

* A reduction in carbohydrate degradation and enhanced carbohydrate complex formation (with DSDE) by the co-distillation of water with injected vapors of the co-distillation solvent, which results in a reduction in cycle time caused by more intimate mass transport. Water removal is an important rate determining factor here, and the sparge system removes the water more rapidly by providing more intimate contact of the co-distillation solvent with the reaction mass.

* A reduction in the time that carbohydrate is at elevated temperature.

* Improved product quality

* Improved reaction selectivity (improved selectivity and improved quality are probably a consequence of better DSDE/sucrose complex formation and decreased carbohydrate degradation rather than any improvement in the acylation reaction)

* Decreased amount of unreacted sucrose

**Claims**

**1.** A process which comprises:

(1) preparing a first reaction mixture comprising sucrose and a polar aprotic solvent;
(2) adding a 1,3-diacyloxy-1,1,3,3-tetra(hydrocarbyl)-distannoxane to said first reaction mixture to form a second reaction mixture;
(3) passing vapors of a second solvent capable of removing water by codistillation through said second reaction mixture, in sufficient quantity and for a period of time sufficient to remove substantially all of the water in said reaction mixture by codistillation, to form thereby a third reaction mixture comprising said polar aprotic solvent and said second solvent, said second reaction mixture being substantially free of water, and;
(4) adding a carboxylic acid anhydride to said third reaction mixture to form a fourth reaction mixture and maintaining said fourth reaction mixture at a temperature and for a period of time sufficient to produce a sucrose-6-ester.

**2.** A process which comprises the steps of:

(1) preparing a first reaction mixture comprising sucrose and a polar aprotic solvent;
(2) passing vapors of a second solvent capable of removing water by codistillation through said first reaction mixture to form thereby a second reaction mixture comprising sucrose, said polar aprotic solvent and said second solvent;
(3) adding a 1,3-diacyloxy-1,1,3,3-tetra(hydrocarbyl)-distannoxane to said second reaction mixture while continuing to pass vapors of said second solvent through said reaction mixture, in such quantity and for a period of time sufficient to remove water by codistillation, to form a third reaction mixture that is substantially free of water; and
(4) adding a carboxylic acid anhydride to said third reaction mixture to form a fourth reaction mixture, and maintaining said fourth reaction mixture at a temperature and for a period of time sufficient to produce a sucrose-6-ester.

**3.** The process of claim 1 or claim 2 wherein the fourth reaction mixture is quenched with water upon the completion of the reaction with the carboxylic acid anhydride.

**4.** The process of any preceding claim wherein the second solvent is selected from the group consisting of hydrocarbons, chlorinated hydrocarbons, ketones, and ethers.

**5.** The process of claim 4 wherein the second solvent is selected from the group consisting of toluene, cyclohexane, n-heptane, and isooctane.

**6.** The process of any preceding claim wherein the 1,3-diacyloxy-1,1,3,3-tetra(hydrocarbyl)distannoxane is selected from the group consisting of 1,3-diacetoxy-1,1,3,3-tetrabutyldistannoxane, 1,3-dibenzoyloxy-1,1,3,3-tetrabutyldistannoxane, 1,3-diacetoxy-1,1,3,3-tetraoctyldistannoxane, and 1,3-dibenzoyloxy-1,1,3,3-tetraoctyldistannoxane,

wherein the polar aprotic solvent is *N,N*-dimethylformamide, and wherein the carboxylic acid anhydride is acetic anhydride or benzoic anhydride.

**7.** The process of any preceding claim wherein the carboxylic acid anhydride is acetic anhydride.

**8.** The process of any of claims 1 to 6 wherein the carboxylic acid anhydride is benzoic anhydride.

**9.** A process which comprises passing vapors of a codistillation solvent capable of removing water by codistillation through a reaction mixture containing (a) a polar aprotic solvent and (b) sucrose, a 1,3-diacyloxy-1,1,3,3-tetra (hydrocarbyl)distannoxane, and the reaction product of sucrose and a 1,3-diacyloxy-1,1,3,3-tetra(hydrocarbyl)distannoxane, in sufficient quantity and for a period of time sufficient to remove substantially all of the water in said reaction mixture by codistillation.

**10.** The process of claim 9 wherein the codistillation solvent is a member selected from the group consisting of hydrocarbons, chlorinated hydrocarbons, ketones, and ethers.

**11.** The process of claim 9 wherein the codistillation solvent is a member selected from the group consisting of toluene, cyclohexane, n-heptane, and isooctane.

**12.** The process of any of claims 9 to 11 wherein the 1,3-diacyloxy-1,1,3,3-tetra (hydrocarbyl) distannoxane is selected from the group consisting of 1,3-diacetoxy-1,1,3,3-tetrabutyldistannoxane, 1,3-dibenzoyloxy-1,1,3,3-tetrabutyldistannoxane, 1,3-diacetoxy-1,1,3,3-tetraoctyldistannoxane, and 1,3-dibenzoyloxy-1,1,3,3-tetraoctyldistannoxane, and wherein the polar aprotic solvent is *N,N*-dimethylformamide.

**Revendications**

**1.** Procédé qui comprend :

(1) la préparation d'un premier mélange de réaction, comprenant du sucrose et un solvant polaire aprotique ;

(2) l'addition d'un 1,3-diacyloxy-1,1,3,3-tétra-(hydrocarbyl)distannoxane audit premier mélange de réaction pour former un deuxième mélange de réaction ;

(3) le passage de vapeurs d'un deuxième solvant capable d'éliminer l'eau par codistillation au travers dudit deuxième mélange de réaction, en une quantité suffisante et pendant une période de temps suffisante pour éliminer substantiellement toute l'eau dans ledit mélange de réaction par codistillation, pour ainsi former un troisième mélange de réaction comprenant ledit solvant polaire aprotique et ledit deuxième solvant, ledit deuxième mélange de réaction étant substantiellement exempt d'eau, et

(4) l'addition d'un anhydride d'acide carboxylique audit troisième mélange de réaction pour former un quatrième mélange de réaction et en maintenant ledit quatrième mélange de réaction à une température et pendant une période de temps suffisante pour produire un sucrose-6-ester.

**2.** Procédé qui comprend :

(1) la préparation d'un premier mélange de réaction, comprenant du sucrose et un solvant polaire aprotique ;

(2) le passage de vapeurs d'un deuxième solvant capable d'éliminer l'eau par codistillation au travers dudit premier mélange de réaction, pour ainsi former un deuxième mélange de réaction comprenant le sucrose, ledit solvant polaire aprotique et ledit deuxième solvant ;

(3) l'addition d'un 1,3-diacyloxy-1,1,3,3-tétra(hydrocarbyl)distannoxane audit deuxième mélange de réaction en continuant à faire passer des vapeurs dudit deuxième solvant dans ledit mélange de réaction, en une quantité telle et pendant une période suffisante pour éliminer l'eau par codistillation, pour former un troisième mélange de réaction, qui est substantiellement exempt d'eau, et

(4) l'addition d'un anhydride d'acide carboxylique audit troisième mélange de réaction pour former un quatrième

mélange de réaction et en maintenant ledit quatrième mélange de réaction à une température et pendant une période de temps suffisante pour produire un sucrose-6-ester.

3. Procédé selon la revendication 1 ou la revendication 2, dans lequel le quatrième mélange de réaction est trempé à l'eau lors de l'achèvement de la réaction avec l'anhydride d'acide carboxylique.

4. Procédé selon l'une quelconque des revendications précédentes, dans lequel le deuxième solvant est choisi parmi le groupe consistant en des hydrocarbures, des hydrocarbures chlorés, des cétones et des éthers.

5. procédé selon la revendication 4, dans lequel le deuxième solvant est choisi parmi le groupe consistant en le toluène, le cyclohexane, le n-heptane et l'isooctane.

6. Procédé selon l'une quelconque des revendications précédentes, dans lequel le 1,3-diacyloxy-1,1,3,3-tétra(hydrocarbyl)distannoxane est choisi parmi le groupe consistant en le 1,3-diacétoxy-1,1,3,3-tétrabutyldistannoxane, le 1,3-dibenzoyloxy-1,1,3,3-tétrabutyldistannoxane, le 1,3-diacétoxy-1,1,3,3-tétraoctyl-distannoxane et le 1,3-dibenzoyloxy-1,1,3,3-tétraoctyldistannoxane, dans lequel le solvant polaire aprotique est le N,N-diméthylformamide et dans lequel l'anhydride d'acide carboxylique est l'anhydride acétique ou l'anhydride benzoïque.

7. Procédé selon l'une quelconque des revendica-tions précédentes, dans lequel l'anhydride d'acide carboxylique est l'anhydride acétique.

8. Procédé selon l'une quelconque des revendica-tions 1 à 6, dans lequel l'anhydride d'acide carbo-xylique est l'anhydride benzoïque.

9. Procédé qui comprend le passage de vapeurs d'un solvant de codistillation capable d'éliminer l'eau par codistillation au travers d'un mélange de réaction contenant (a) un solvant polaire aprotique, et (b) du sucrose, un 1,3-diacyloxy-1,1,3,3-tétra(hydrocarbyl)distannoxane, et le produit de réaction du sucrose et d'un 1,3-diacyloxy-1,1,3,3-tétra(hydrocarbyl)distannoxane, en une quantité suffisante et pendant une période suffisante pour éliminer substantiellement toute l'eau dans ledit mélange de réaction par codistillation.

10. Procédé selon la revendication 9, dans lequel le solvant de codistillation est un membre sélectionné parmi le groupe consistant en des hydrocarbures, des hydrocarbures chlorés, des cétones et des éthers.

11. Procédé selon la revendication 9, dans lequel le solvant de codistillation est un membre choisi parmi le groupe consistant en le toluène, le cyclohexane, le n-heptane et l'isooctane.

12. Procédé selon l'une quelconque des revendications 9 à 11, dans lequel le 1,3-diacyloxy-1,1,3,3-tétra(hydrocarbyl) distannoxane est choisi parmi le groupe consistant en le 1,3-diacétoxy-1,1,3,3-tétrabutyldistannoxane, le I,3-dibenzoyloxy-1,1,3,3-tétrabutyldistannoxane, le 1,3-diacétoxy-1,1,3,3-tétraoctyldistannoxane et le 1,3-dibenzoyloxy-1,1,3,3-tétraoctyldistannoxane, dans lequel le solvant polaire aprotique est le N,N-diméthylformamide.

**Patentansprüche**

1. Verfahren, welches umfaßt:

(1) Herstellen einer ersten Reaktionsmischung, welche Sucrose und ein polares aprotisches Lösungsmittel umfaßt;

(2) Zufügen von 1,3-Diacyloxy-1,1,3,3-tetra(hydrocarbyl)-distannoxan zu der ersten Reaktionsmischung, um eine zweite Reaktionsmischung zu bilden;

(3) Leiten von Dämpfen eines zweiten Lösungsmittels, das Wasser durch Codestillation entfernen kann, durch die zweite Reaktionsmischung, in ausreichender Menge und für eine Zeitdauer, die ausreichend ist, um im wesentlichen das gesamte Wasser in der Reaktionsmischung durch Codestillation zu entfernen, um dadurch eine dritte Reaktionsmischung zu bilden, welche das polare aprotische Lösungsmittel und das zweite Lösungsmittel umfaßt, wobei die zweite Reaktionsmischung im wesentlichen frei von Wasser ist; und

(4) Zufügen eines Carbonsäureanhydrids zu der dritten Reaktionsmischung, um eine vierte Reaktionsmischung zu bilden, und Halten der vierten Reaktionsmischung bei einer Temperatur und für eine Zeitdauer, die ausreichend sind, um einen Sucrose-6-Ester herzustellen.

**2.** Verfahren, welches die Schritte umfaßt:

(1) Herstellen einer ersten Reaktionsmischung, welche Sucrose und ein polares aprotisches Lösungsmittel umfaßt;

(2) Leiten von Dämpfen eines zweiten Lösungsmittels, das Wasser durch Codestillation entfernen kann, durch die erste Reaktionsmischung, um dadurch eine zweite Reaktionsmischung zu bilden, welche Sucrose, das polare aprotische Lösungsmittel und das zweite Lösungsmittel umfaßt;

(3) Zurügen von 1,3-Diacyloxy-1,1,3,3-tetra(hydroxycarbyl)-distannoxan zu der zweiten Reaktionsmischung, während das Leiten von Dämpfen des zweiten Lösungsmittels durch die Reaktionsmischung fortgeführt wird, in solcher Menge und für eine Zeitdauer, die ausreichend ist, um Wasser durch Codestillation zu entfernen, um eine dritte Reaktionsmischung zu bilden, die im wesentlichen frei von Wasser ist;

(4) Zufügen eines Carbonsäureanhydrids zu der dritten Reaktionsmischung, um eine vierte Reaktionsmischung zu bilden, und Halten der vierten Reaktionsmischung bei einer Temperatur und für eine Zeitdauer, die ausreichend sind, um einen Sucrose-6-Ester herzustellen.

**3.** Verfahren nach Anspruch 1 oder Anspruch 2, **dadurch gekennzeichnet, daß** die vierte Reaktionsmischung bei Vollständigkeit der Reaktion mit dem Carbonsäureanhydrid mit Wasser gequencht wird.

**4.** Verfahren nach einem der vorangehenden Ansprüche, **dadurch gekennzeichnet, daß** das zweite Lösungsmittel ausgewählt wird aus der Gruppe bestehend aus Kohlenwasserstoffen, chlorierten Kohlenwasserstoffen, Ketonen und Ethern.

**5.** Verfahren nach Anspruch 4, **dadurch gekennzeichnet, daß** das zweite Lösungsmittel ausgewählt wird aus der Gruppe bestehend aus Toluol, Cyclohexan, n-Heptan und Isooctan.

**6.** Verfahren nach einem der vorangehenden Ansprüche, **dadurch gekennzeichnet, daß** das 1,3-Diacyloxy-1,1,3,3-tetra(hydroxycarbyl)distannoxan ausgewählt wird aus der Gruppe bestehend aus 1,3-Diacetoxy-1,1,3,3-tetrabutyldistannoxan, 1,3-Dibenzoyloxy-1,1,3,3-tetrabutyldistannoxan, 1,3-Diacetoxy-1,1,3,3-tetraoctyldistannoxan und 1,3-Dibenzoyloxy-1,1,3,3-tetraoctyldistannoxan, wobei das polare aprotische Lösungsmittel N,N-Dimethylformamid ist, und wobei das Carbonsäureanhydrid Essigsäureanhydrid oder Benzoesäureanhydrid ist.

**7.** Verfahren nach einem der vorangehenden Ansprüche, **dadurch gekennzeichnet, daß** das Carbonsäureanhydrid Essigsäureanhydrid ist.

**8.** Verfahren nach einem der Ansprüche 1 bis 6, **dadurch gekennzeichnet, daß** das Carbonsäureanhydrid Benzoesäureanhydrid ist.

**9.** Verfahren, welches umfaßt ein Leiten von Dämpfen eines Codestillationslösungsmittels, das Wasser durch Codestillation entfernen kann, durch eine Reaktionsmischung, welche enthält (a) ein polares aprotisches Lösungmsittel und (b) Sucrose, ein 1,3-Diacyloxy-1,1,3,3-tetra(hydrocarbyl)distannoxan und das Reaktionsprodukt von Sucrose und einem 1,3-Diacyloxy-1,1,3,3-tetra(hydrocarbyl)distannoxan, in ausreichender Menge und für eine Zeitdauer, die ausreichend ist, um im wesentlichen das gesamte Wasser in der Reaktionsmischung durch Codestillation zu entfernen.

**10.** Verfahren nach Anspruch 9, **dadurch gekennzeichnet, daß** das Codestillationslösungsmittel ein Mitglied ist, das ausgewählt wird aus der Gruppe bestehend aus Kohlenwasserstoffen, chlorierten Kohlenwasserstoffen, Ketonen und Ethern.

**11.** Verfahren nach Anspruch 9, **dadurch gekennzeichnet, daß** das Codestillationslösungsmittel ein Mitglied ist, das ausgewählt wird aus der Gruppe bestehend aus Toluol, Cyclohexan, n-Heptan und Isooctan.

**12.** Verfahren nach einem der Ansprüche 9 bis 11, **dadurch gekennzeichnet, daß** das 1,3-Diacyloxy-1,1,3,3-tetra (hydrocarbyl)distannoxan ausgewählt wird aus der Gruppe bestehend aus 1,3-Diacetoxy-1,1,3,3-tetrabutyldistannoxan, 1,3-Dibenzoyloxy-1,1,3,3-tetrabutyldistannoxan, 1,3-Diacetoxy-1,1,3,3-tetraoctyldistannoxan und 1,3-Dibenzoyloxy-1,1,3,3-tetraoctyldistannoxan, und wobei das polare aprotische Lösungsmittel N,N-Dimethylformamid ist.